(19) Europäisches Patentamt
European Patent Office
Office européen des brevets

(11) Veröffentlichungsnummer: **0 011 266**
**B1**

(12) # EUROPÄISCHE PATENTSCHRIFT

(45) Veröffentlichungstag der Patentschrift: **07.10.81**

(51) Int. Cl.³: **C 07 C 135/00** // C07D239/30

(21) Anmeldenummer: **79104442.3**

(22) Anmeldetag: **18.11.78**

(54) **(2-Cyanethyl)-Isocyaniddichlorid und ein Verfahren zu dessen Herstellung.**

(30) Priorität: **29.11.77 DE 2753204**

(43) Veröffentlichungstag der Anmeldung: **28.05.80 Patentblatt 80/11**

(45) Bekanntmachung des Hinweises auf die Patenterteilung: **07.10.81 Patentblatt 81/40**

(84) Benannte Vertragsstaaten: **BE CH DE FR GB**

(56) Entgegenhaltungen:
**DE-B-1 094 737**
**DE-A-2 458 826**
**FR-A-976 959**
**US-A-3 932 507**

(73) Patentinhaber: **BAYER AG, Zentralbereich Patente, Marken und Lizenzen, D-5090 Leverkusen 1, Bayerwerk (DE)**

(72) Erfinder: **Beck, Gunther, Dr., Am Mittelberg 19, D-5090 Leverkusen (DE)**
Erfinder: **Dankert, Gerhard, Dr., Arthur-Hantzsch-Strasse 44, D-5000 Köln 80 (DE)**
Erfinder: **Döring, Fritz, Am Hang 13, D-5068 Odenthal (DE)**

EP 0 011 266 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# (2-Cyanethyl)-isocyaniddichlorid und ein Verfahren zu dessen Herstellung

Gegenstand der vorliegenden Erfindung ist die neue Verbindung (2-Cyanethyl)-isocyaniddichlorid der Formel

$$NC\text{–}CH_2\text{–}CH_2\text{–}N = CCl_2 \qquad (I)$$

sowie ein Verfahren zu ihrer Herstellung.

Die vorgenannte Verbindung kann dadurch hergestellt werden, dass man das in bekannter Weise (Französische Patentschrift 976 959) z.B. aus Formamid und Acrylnitril zugängliche (2-Cyanethyl)-formamid (II) gemäss der Reaktionsgleichung

$$\underset{(II)}{NC\text{–}CH_2\text{–}CH_2\text{–}NH\text{–}CHO} + Cl_2 \rightarrow$$

$$\underset{(I)}{NC\text{–}CH_2\text{–}CH_2\text{–}N = CCl_2} + SO_2 + 2\ HCl$$

bei Temperaturen von 40–80 °C, vorzugsweise 50–75 °C, mit Thionylchlorid und Chlor, bzw. einer unter den Reaktionsbedingungen Chlor abspaltenden Verbindung, vorzugsweise Sulfurylchlorid, umsetzt.

Besonders gute Ausbeuten (etwa 90%) und hohe Reinheiten (bis zu 98%) an (I) erhält man, wenn man (2-Cyanethyl)-formamid (II) in eine Mischung aus Thionylchlorid und Sulfurylchlorid bzw. Chlor einträgt, bei der das molare Verhältnis Thionylchlorid : Sulfurylchlorid bzw. Chlor wesentlich grösser als 1:1, im allgemeinen etwa 3:1 bis 10:1 beträgt. Ein noch höheres Molverhältnis ist möglich, bringt aber keine wesentlichen Vorteile.

Das Molverhältnis Chlor bzw. Chlor abspaltende Verbindung, insbesondere Sulfurylchlorid, zu (II) sollte zur Erzielung einer möglichst hohen Ausbeute an (I) mindestens 1:1 betragen. In der Praxis wendet man Molverhältnisse zwischen 1:1 und 2,5:1 an. Ein noch höheres Molverhältnis ist möglich, bringt aber keine Vorteile.

Es kann zweckmässig sein, das (2-Cyanäthyl)-formamid (II) bei Temperaturen in das Chlorierungsgemisch einzutragen, die unterhalb 40–80 °C, vorzugsweise 50–75 °C, liegen, beispielsweise zwischen 20 und 35 °C, und das Reaktionsgemisch erst dann aufzuheizen, wenn nach erfolgter Zugabe von (II) die erste, leicht exotherme Reaktion abgeklungen ist.

Die Hauptreaktion findet zwischen 50 und 75 °C statt und ist im allgemeinen nach spätestens zwei Stunden beendet.

Überschüssiges Chlorierungsmittel, insbesondere das überschüssige Thionylchlorid, kann nach dem Abdestillieren vom wesentlich schwerer flüchtigen (2-Cyanäthyl)-isocyaniddichlorid (I) (Kp 107 °C, 17 mbar) für weitere Ansätze verwendet werden. Selbstverständlich kann man auch kontinuierlich arbeiten.

Das nach dem Abziehen des überschüssigen Thionylchlorids und gegebenenfalls überschüssigen Chlorierungsmittels, insbesondere Sulfurylchlorid, hinterbleibende (2-Cyanethyl)-isocyaniddichlorid (I) ist im allgemeinen bereits von hoher Reinheit (>90%) und kann direkt in die anschliessend beschriebene Gasphasenchlorierung eingesetzt werden. Durch eine einfache Destillation, bei der nur geringfügige Mengen undestillierbarer Rückstände hinterbleiben, erhält man bei $Kp_{13}$ 107 °C ein gaschromatographisch fast 98% reines (I) in etwa 90%iger Ausbeute.

Das erfindungsgemässe (2-Cyanethyl)-isocyaniddichlorid findet beispielsweise Verwendung zur Herstellung von Tetrachlorpyrimidin, das man daraus durch Umsetzung mit Chlor in der Gasphase bei 200–650 °C erhält.

Dadurch wird ein erfinderischer Weg zur Verbindung Tetrachlorpyrimidin eröffnet. Tetrachlorpyrimidin findet entweder als solches, z.B. als gegen pflanzliche Pilze wirksamer Stoff (siehe z.B. US-PS 3 227 617) oder auch als Zwischenprodukt bei der Herstellung wertvoller Reaktivfarbstoffe Verwendung (siehe dazu z.B. BE-PS 578 933).

Das Verfahren, aus (2-Cyanethyl)-isocyaniddichlorid mit Chlor Tetrachlorpyrimidin herzustellen, ist aus dem im folgenden genannten Grund erfinderisch.

In Angewandte Chemie, 82. Jahrgang, Nr. 2, Seiten 78–79, wird die Umsetzung von $\alpha$-dihalogenierten Isocyanid-dichloriden mit Nitrilen zu chlorierten Pyrimidinen beschrieben, wobei die Nitrile in $\alpha$-Stellung zwei Wasserstoffatome besitzen müssen und in Gegenwart von Lewis-Säure gearbeitet werden muss. Dieses Verfahren – bei dem im übrigen die im Vergleich zu 2-Cyanethylisocyanid-dichlorid wesentlich schwieriger zugänglichen $\alpha$-dihalogenierten Isocyanid-dichloride eingesetzt werden müssen – ist grundsätzlich verschieden von dem beanspruchten Verfahren und legt dieses in keiner Weise nahe.

Beispiel 1

Herstellung von (2-Cyanethyl)-isocyaniddichlorid:

a) mit Thionylchlorid/Sulfurylchlorid:

In eine gerührte Mischung aus 200 ml (2,48 Mol) Sulfurylchlorid und 1200 ml (16,5 Mol) Thionylchlorid wurden bei 22 °C ohne Kühlung im Verlauf einer halben Stunde 98 g (1,0 Mol) (2-Cyanäthyl)-formamid getropft, wobei die Innentemperatur allmählich auf 34 °C anstieg; gleichzeitig wurde ein Niederschlag gebildet. Es wurde noch 20 Minuten nachgerührt, wobei die Temperatur auf 30 °C sank. Anschliessend wurde 2 Stunden bei einer Ölbadtemperatur von 90 °C unter Rückfluss gerührt. Bei 50–55 °C ging der Niederschlag unter Gasentwicklung in Lösung. Am Ende des zweistündigen Erhitzens ist die Rückflusstemperatur bis auf 70 °C angestiegen. Nach Abziehen von $SOCl_2$ im Vakuum wurden bei Kp 107 °C 17 mbar 138 g (2-Cyanäthyl)-isocyaniddichlorid in einer gaschromatographisch ermittelten Reinheit von 97,7% (entsprechend 89% der Theorie) erhalten.

b)mit Thionylchlorid/Chlor:

In einer 4-Liter-Rührapparatur werden 2200 g (18,48 Mol) Thionylchlorid vorgelegt und bei 55 °C im Verlauf von 4 Stunden gleichzeitig 453 g (4,62 Mol) (2-Cyanäthyl)-formamid zugetropft und 365 g (5,14 Mol) Chlor eingeleitet. Nach etwa 30 minütigem Nachrühren bei 55–60 °C ist die Gasentwicklung weitgehend beendet. Die Aufarbeitung erfolgt durch Destillation. Man erhält bei Kp 100–110 °C 16 mbar 663 g (2-Cyanäthyl)-isocyaniddichlorid in einer gaschromatographisch ermittelten Reinheit von 95% (entsprechend 95% der Theorie).

Beispiel 2

In einen als Verdampfer fungierenden 500 ml-Dreihalskolben, der durch ein Ölbad von 240 °C beheizt ist und mit einer durch eine elektrische Heizbandage auf 240 °C beheizten Brücke versehen ist, werden im Verlauf von 95 Minuten gleichzeitig 123 g (0,815 Mol) (2-Cyanäthyl)-isocyaniddichlorid und 231 g (3,25 Mol) Chlor mittels Dosierungspumpe bzw. Strömungsmesser eingebracht. An die Brücke schliesst sich ein vertikal aufgehängtes, durch elektrische Wicklung auf 240 °C beheiztes Strömungsrohr (Länge 300 mm, Durchmesser 30 mm) an, das mit Aktivkohle von einem Teilchendurchmesser von 2–3 mm gefüllt ist (Kontaktvolumen 185 ml) und in dem das im Verdampfer erzeugte Gasgemisch zur Umsetzung gelangt. Zur Abfuhr der Reaktionswärme ist das Strömungsrohr nach Art eines Liebigkühlers von einem Aussenmantel umgeben, durch den Inertgas geleitet wird.

Ein koaxial in das Strömungsrohr eingebrachtes Thermoelement kontrolliert die Kontakttemperatur von 240 °C. Als Vorlage dient ein durch ein Eis-Wasser-Bad gekühlter 2 1-Zweihalskolben, in dem das Reaktionsprodukt kondensiert wird und durch dessen zweiten Stutzen der gebildete Chlorwasserstoff und gegebenenfalls überschüssiges Chlor abgeleitet werden.

Nach beendeter Reaktion wird das Kondensat bei 50 °C im Wasserstrahlvakuum kurz entgast. Auswaage: 175 g. Die gaschromatographische Analyse ergab einen Gehalt von 96% (entsprechend 168 g) Tetrachlorpyrimidin; das entspricht einer Ausbeute von 94,6% der Theorie.

## Patentansprüche

1. (2-Cyanethyl)-isocyaniddichlorid.
2. Verfahren zur Herstellung von (2-Cyanethyl)-isocyaniddichlorid, dadurch gekennzeichnet, dass man (2-Cyanethyl)-formamid bei Temperaturen von 40 bis 80 °C mit Thionylchlorid und Chlor oder einer unter den Reaktionsbedingungen Chlor abspaltenden Verbindung umsetzt.

## Claims

1. 2-Cyanoethyl isocyanide dichloride.
2. Process for the preparation of 2-cyanoethyl isocyanide dichloride, characterised in that 2-cyanoethyl-formamide is reacted with thionyl chloride and chlorine, or a compound which splits off chlorine under the reaction conditions, at temperatures from 40 to 80 °C.

## Revendications

1. Le dichlorure de l'isocyanure de 2-cyanéthyle.
2. Procédé de préparation du dichlorure de l'isocyanure de 2-cyanéthyle, caractérisé en ce que l'on fait réagir le (2-cyanéthyl)-formamide à des températures de 40 à 80 °C avec le chlorure de thionyle et le chlore ou un composé libérant du chlore dans les conditions de la réaction.